# EUROPÄISCHE PATENTSCHRIFT

(11) **EP 4 193 970 B1**
(45) Veröffentlichungstag und Bekanntmachung des Hinweises auf die Patenterteilung: **12.02.2025**
(21) Anmeldenummer: 22212115.4
(22) Anmeldetag: 08.12.2022
(51) Int. Cl.: A61F 5/01

(54) **ORTHOPÄDIETECHNISCHE EINRICHTUNG UND VERFAHREN ZUM HERSTELLEN**
ORTHOPEDIC DEVICE AND METHOD FOR PRODUCING SAME
DISPOSITIF ORTHOPÉDIQUE ET PROCÉDÉ DE FABRICATION

(30) Priorität: 13.12.2021 DE 102021006129
(43) Veröffentlichungstag der Anmeldung: 14.06.2023
(73) Patentinhaber: Plus Medica OT GmbH, 40549 Düsseldorf (DE)
(72) Erfinder: PIEPER, Tobias, 40549 Düsseldorf (DE); HÜLK, Alexander, 40549 Düsseldorf (DE)
(74) Vertreter: Gramm, Lins & Partner Patent- und Rechtsanwälte PartGmbB

(56) Entgegenhaltungen:
- WO-A1-2013/156351
- US-A1- 2015 150 709
- US-A1- 2015 305 912

## Beschreibung

Die Erfindung betrifft eine orthopädietechnische Einrichtung mit einem formstabilen Element zum Positionieren an einem Körperteil und einem Gurt zum Befestigen des formstabilen Elementes an dem Körperteil, wobei der Gurt durch eine Umlenköse geführt ist. Die Erfindung betrifft zudem ein Verfahren zum Herstellen einer solchen orthopädietechnischen Einrichtung.

Orthopädietechnische Einrichtungen sind beispielsweise Orthesen oder Prothesen. Im Rahmen der vorliegenden Erfindung werden jedoch auch Unterstützungsvorrichtungen, die Körperteile oder den Träger der Vorrichtung unterstützen und beispielsweise Müdigkeit oder Erschöpfung verhindern oder heraus zögern sollen, unter orthopädietechnischen Einrichtungen verstanden. Die orthopädietechnische Einrichtung verfügt über ein formstabiles Element, das an dem jeweiligen Körperteil mittels des Gurtes befestigt wird. Das formstabile Element kann beispielsweise eine Schiene oder eine Schale sein. Das formstabile Element kann ein Standardelement sein, das nicht individuell an das Körperteil angepasst ist. Alternativ dazu kann das formstabile Element auch individuell für den Träger an das jeweilige Körperteil des Trägers angepasst sein.

Das formstabile Element kann aus einem Kunststoff, beispielsweise einem faserverstärkten Kunststoff oder aus Metall hergestellt sein. Unter einem Gurt wird vorliegend ein flexibles Element verstanden, das vorzugsweise um das Körperteil des Trägers, an dem das formstabile Element befestigt werden soll, herumgelegt wird. Der Gurt kann elastisch oder unelastisch ausgebildet sein. Eine Umlenköse ist im Sinne der vorliegenden Erfindung jedes Element, um das der Gurt herumgelegt wird, um seine Richtung zu ändern. Dabei kann es sich um einen Ring, beispielsweise einen D-Ring oder einen O-Ring handeln. Auch Schlaufen oder einseitig offene Ösen, wie sie beispielsweise von der Schnürung von Wanderschuhen bekannt sind, werden als Umlenkösen angesehen.

Aus dem Stand der Technik sind entsprechende orthopädietechnische Einrichtungen bekannt, deren formstabiles Element über einen oder mehrere Gurte am Körperteil des Trägers befestigt wird. In bevorzugten Ausgestaltungen ist der Gurt mit einem Ende an dem formstabilen Element befestigt. Er wird zumindest teilweise um das Körperteil des Trägers herumgelegt und durch die Umlenköse geführt. Anschließend wird der Gurt in die entgegengesetzte Richtung zumindest teilweise um das Körperteil herumgeführt und festgelegt. Der Gurt kann dabei auf sich selber festgelegt werden. Die eine Seite des Gurtes ist in diesem Fall vorzugsweise mit einem Klettverschluss-Element versehen. Die entgegengesetzte Seite des Gurtes verfügt über das Gegenelement, sodass der Gurt durch das Schließen des Klettverschluss auf sich selbst festgelegt werden kann. Alternativ oder zusätzlich dazu kann das Ende des Gurtes, nachdem es durch die Umlenköse geführt wird, auch am formstabilen Element der orthopädietechnischen Einrichtung oder einem anderen Bauteil oder Element der orthopädietechnischen Einrichtung festgelegt sein.

Durch die Positionierung insbesondere der Umlenköse wird der Verlauf des Gurtes im angelegten Zustand der orthopädietechnischen Einrichtung folglich stark mitbestimmt. Ein nicht optimaler Sitz und Verlauf des Gurtes kann jedoch zu Druckstellen und Schmerzen beim Träger, zumindest jedoch zu einem unbequemen Tragegefühl führen. Insbesondere wenn es sich bei dem formstabilen Element um ein Standardbauteil handelt, welches nicht individuell für den Träger hergestellt ist, ist der optimale Verlauf des Gurtes oftmals nicht im Detail vorherzusagen. Selbst bei individuell hergestellten Einzelstücken, die als formstabiles Element verwendet werden, ist diese Vorhersage oft schwierig, da das individuelle Tragegefühl des Trägers nicht oder nur schwer vorhergesagt werden kann. Die Position und der Verlauf des Gurtes und/oder der Umlenköse ist jedoch nach der Fertigstellung der orthopädietechnischen Einrichtung gemäß dem Stand der Technik nicht zu ändern. Der Träger muss folglich entweder mit dem nicht optimalen Sitz vorlieb nehmen oder es muss eine neue orthopädietechnische Einrichtung erstellt werden. Dies ist kosten- und zeitintensiv.

WO 2013/156351 A1 offenbart eine Spannvorrichtung zur Anwendung im orthopädischen Sektor mit einem starren Körper, wobei die Spannvorrichtung über eine Haltevorrichtung und einem Band an einem Körperteil festlegbar ist. Die Haltevorrichtung hat dabei ein Verbindungselement und ein sich verdickendes Ende, wobei das Verbindungselement und das verdickende Ende in eine korrespondierende Ausnehmung einer Befestigungseinrichtung geführt werden kann.

Der Erfindung liegt die Aufgabe zugrunde, die Nachteile aus dem Stand der Technik zu beheben oder zumindest zu lindern.

Die Erfindung löst die gestellte Aufgabe durch eine orthopädietechnische Einrichtung gemäß dem Oberbegriff des Anspruchs 1, die sich dadurch auszeichnet, dass das formstabile Element wenigstens eine Befestigungseinrichtung mit einer Durchführung mit einer Längsrichtung aufweist, entlang der sich ein Verbindungselement durch die Durchführung erstreckt, an dem die Umlenköse befestigt ist, wobei das Verbindungselement ein erstes Ende aufweist, an dem ein erster Anker derart ausgebildet ist, dass das erste Ende mit dem ersten Anker nicht entlang der Längsrichtung durch die Durchführung gezogen werden kann.

Wird das Verbindungselement aus der Durchführung entfernt, in dem es soweit durch die Durchführung hindurch bewegt wird, dass es diese vollständig verlässt, wird im Rahmen der vorliegenden Erfindung davon gesprochen, dass das Verbindungselement durch die Durchführung "gezogen" wird. Dadurch wird lediglich die Bewegung, nicht jedoch die Richtung der aufgebrachten Kraft, die für diese Bewegung verantwortlich ist, beschrieben. Selbst verständlich ist es auch möglich, eine Druckkraft aufzubringen, die diese Bewegung des Verbindungselementes durch die Durchführung hervorruft. Auch dann wird im Rahmen der vorliegenden Erfindung davon gesprochen, dass das Verbindungselement durch die Durchführung "gezogen" wird.

Vorzugsweise ist das Verbindungselement dennoch lösbar über die Befestigungseinrichtung an dem formstabilen Element der orthopädietechnischen Einrichtung befestigt. Das Verbindungselement ist beispielsweise ein Seil, ein Draht oder eine Schnur. Das Verbindungselement verfügt über eine Länge, durch die beispielsweise ein Abstand der Umlenköse von der Befestigungseinrichtung bestimmt wird. Dabei ist es nicht zwangsläufig notwendig, dass dieser Abstand der Länge des Verbindungselementes entspricht. Es gilt jedoch, dass ein längeres Verbindungselement auch einen größeren Abstand der Umlenköse von der Befestigungseinrichtung zur Folge hat.

In einer bevorzugten Ausgestaltung ist der erste Anker lösbar an dem ersten Ende des Verbindungselementes angeordnet. Der erste Anker kann beispielsweise in Form einer Hülse ausgebildet sein, die ein Innengewinde aufweist, das mit einem Außengewinde korrespondierend ausgebildet ist, das am ersten Ende des Verbindungselementes angeordnet ist. Auf diese Weise ist es besonders einfach möglich, den ersten Anker vom ersten Ende des Verbindungselementes zu lösen. In einer bevorzugten Ausgestaltung weist der erste Anker einen größeren Querschnitt als das Verbindungselement auf. Dabei ist der Durchmesser des ersten Ankers vorzugsweise größer als der Durchmesser der Durchführung, sodass die Befestigungseinrichtung zwar durch die Durchführung bewegt und innerhalb dieser verschoben werden kann, jedoch nicht entlang der Längsrichtung durch die Durchführung gezogen und so vom formstabilen Element der orthopädietechnischen Einrichtung entfernt werden kann. Dies verhindert in diesem Fall der erste Anker, der einen zu großen Querschnitt oder Durchmesser aufweist, um ihn durch die Durchführungen durchzuziehen.

Wird der erste Anker von dem ersten Ende des Verbindungselementes entfernt kann das Verbindungselement und das erste Ende des Verbindungselementes entlang der Längsrichtung durch die Durchführung gezogen und das Verbindungselement so vom formstabilen Element getrennt und entfernt werden. Auf diese Weise ist es beispielsweise möglich, ein Verbindungselement gegen ein anderes Verbindungselement auszutauschen, das beispielsweise eine andere Länge oder eine andere Art von Umlenköse aufweist.

Alternativ oder zusätzlich dazu verfügt die Durchführung über einen Abschnitt, in dem der erste Anker zumindest teilweise, vorzugsweise jedoch vollständig, aufnehmbar ist. In einer bevorzugten Ausgestaltung ist dieser Abschnitt mit einem größeren Querschnitt als der verbleibende Teil der Durchführung ausgebildet, wobei dieser größere Querschnitt vorzugsweise dem Querschnitt und Durchmesser des ersten Ankers entspricht. Im angelegten Zustand der orthopädietechnischen Einrichtung wird durch den Gurt über die Umlenköse eine Zugkraft auch auf das Verbindungselement ausgeübt. Diese Zugkraft wirkt vorzugsweise in die Richtung, in der der erste Anker nicht durch die Durchführung gezogen werden kann. Um beispielsweise zu verhindern, dass ein lösbarer Anker versehentlich vom ersten Ende des Verbindungselementes gelöst wird, kann dieser in dem Abschnitt der Durchführung aufgenommen werden. Auch aus ästhetischen und optischen Gesichtspunkten kann es von Vorteil sein, den ersten Anker in dem Abschnitt der Durchführung anzuordnen und so aus dem Gesichtsfeld des Benutzers zu entfernen.

In einer bevorzugten Ausgestaltung verfügt die Durchführung über einen Schlitz, dessen Breite größer als der Durchmesser des Verbindungselementes und kleiner als der Durchmesser des Ankers ist. Vorzugsweise erstreckt sich der Schlitz über die gesamte Längsrichtung der Durchführung. Wird in dieser Ausgestaltung eine Zugkraft auf das Verbindungselement ausgeübt, wie dies beispielsweise im angelegten Zustand der orthopädietechnischen Einrichtung durch den Gurt geschieht, wird das Verbindungselement dieser Kraft folgend soweit wie möglich durch die Durchführung hindurch bewegt. Diese Bewegung endet, wenn der erste Anker nicht mehr weiter durch die Durchführung hindurch bewegt werden kann, weil er mit seiner Stirnfläche an einer entsprechenden Stirnfläche der Befestigungseinrichtung anliegt. Vorzugsweise befindet sich der erste Anker dann in dem Abschnitt der Durchführung, in dem er aufnehmbar ist. Das Verbindungselement an dessen erstem Ende sich der erste Anker befindet kann in dieser Position nicht aus der Durchführung entfernt werden. Das Verbindungselement kann nicht entlang der Längsrichtung der Durchführung durch diese Durchführung gezogen werden, da dies durch den ersten Anker verhindert wird. Das Verbindungselement kann zudem nicht durch den Schlitz der Durchführung entfernt werden, da dessen Breite zwar größer als der Durchmesser des Verbindungselementes ist, die Breite jedoch zu klein ist, um den sich in der Durchführung befindenden ersten Anker durch den Schlitz zu bewegen.

Bei dieser Ausgestaltung ist es jedoch nicht notwendig, den ersten Anker von dem ersten Ende des Verbindungselementes zu entfernen, um das Verbindungselement vom formstabilen Element des Körperteils zu entfernen. Vielmehr ist es ausreichend, das Verbindungselement soweit entlang der Längsrichtung durch die Durchführung zu bewegen, dass der erste Anker nicht mehr in einem Abschnitt der Durchführung aufgenommen wird. In diesem Zustand erstreckt sich nur noch das Verbindungselement durch die Durchführung. Dessen Durchmesser ist kleiner als die Breite des Schlitzes, sodass das Verbindungselement zwar nicht entlang der Längsrichtung aus der Durchführung entfernt werden kann, jedoch durch den Schlitz der Durchführung diese verlassen kann. In dieser Ausgestaltung ist es folglich auch mit einem nicht lösbaren ersten Anker möglich, dass Verbindungselement aus der Durchführung und somit vom formstabilen Element der orthopädietechnischen Einrichtung zu entfernen.

Vorzugsweise verfügt das Verbindungselement über ein zweites Ende, an dem ein zweiter Anker ausgebildet ist. Das zweite Ende ist vorzugsweise dem ersten Ende gegenüber angeordnet.

In einer bevorzugten Ausgestaltung verfügt die Befestigungseinrichtung über eine zweite Durchführung mit einer Längsrichtung, entlang der sich das Verbindungselement erstreckt, wobei der zweite Anker derart ausgebildet ist, dass das zweite Ende mit dem zweiten Anker nicht entlang der Längsrichtung durch die zweite Durchführung gezogen werden kann. Vorzugsweise entspricht die Ausgestaltung der zweiten Durchführung der Ausgestaltung der ersten Durchführung.

Das Verbindungselement erstreckt sich in dieser Ausgestaltung folglich mit beiden Enden durch jeweils eine der beiden Durchführungen. Es bildet somit eine Schlaufe, an der die Umlenköse befestigt oder befestigbar ist. Die Umlenköse kann beispielsweise über einen Kanal verfügen, durch den das Verbindungselement geführt ist. Die beiden Durchführungen der Befestigungseinrichtung verlaufen vorzugsweise nicht parallel zueinander, sondern verlaufen aufeinander zu. Die beiden Anker sind dabei vorzugsweise auf der gleichen Seite der beiden Durchführungen angeordnet. In einer anderen Ausgestaltung bildet ein Anteil des Verbindungselementes die Umlenköse. Dabei handelt es sich um einen Teil des Verbindungelementes, der zwischen den beiden Enden angeordnet ist und der in diesem Fall direkt mit dem Gurt in Kontakt kommt.

Um das Verbindungselement in dieser Ausgestaltung vom formstabilen Element der orthopädietechnischen Einrichtung zu entfernen ist es von Vorteil, wenn sowohl der erste Anker als auch der zweite Anker lösbar an ihrem jeweiligen Ende des Verbindungselementes angeordnet sind. In diesem Fall können sowohl der erste Anker als auch der zweite Anker entfernt werden. Das erste Ende des Verbindungselementes kann danach durch die erste Durchführung gezogen werden. Das zweite Ende kann nach dem Entfernen des zweiten Ankers entlang der Längsrichtung durch die zweite Durchführung gezogen werden. Danach ist das Verbindungselement nicht mehr mit der Befestigungseinrichtung verbunden und kann beispielsweise ausgetauscht werden. Ein Vorteil dieser Ausgestaltung liegt darin, dass die Umlenköse nicht vom Verbindungselement entfernt oder gelöst werden muss.

Vorzugsweise ist die Umlenköse lösbar am Verbindungselement angeordnet. In einer besonders bevorzugten Ausgestaltung ist die Umlenköse derart am Verbindungselement angeordnet, dass sie vom Verbindungselement gelöst werden kann, wenn sich das Verbindungselement durch beide Durchführungen der Befestigungseinrichtung erstreckt. Soll in diesem Fall nicht das Verbindungselement, sondern lediglich die Art oder Größe der Umlenköse verändert werden, sodass die Umlenköse ausgetauscht wird, kann dies geschehen, ohne dass das Verbindungselement vom formstabilen Element der orthopädietechnischen Einrichtung entfernt werden muss.

Um in dieser Ausgestaltung das Verbindungselement austauschen zu können, muss das Verbindungselement vom formstabilen Element der orthopädietechnischen Einrichtung entfernt werden. Dazu ist es ausreichend, einen der beiden Anker vom jeweiligen Ende zu entfernen. Danach kann das jeweilige Ende durch die entsprechende Durchführung gezogen werden. In diesem Zustand kann dann die Umlenköse vom Verbindungselement entfernt werden und das Verbindungselement dann durch die jeweils andere Durchführung gezogen werden. Dabei ist es nicht notwendig, den jeweils anderen Anker zu entfernen, da das Verbindungselement entgegen der Richtung, in der im angelegten Zustand der orthopädietechnischen Einrichtung eine Zugkraft ausgeübt wird, durch die jeweilige Durchführung hindurchgezogen wird.

Vorzugsweise verfügt die zweite Durchführung über einen Schlitz, dessen Breite größer als der Durchmesser des Verbindungselementes und kleiner als der Durchmesser des zweiten Ankers ist. Vorzugsweise verfügt die zweite Durchführung zusätzlich über einen Abschnitt, in dem der zweite Anker zumindest teilweise, vorzugsweise vollständig, aufnehmbar ist. Wird auf das Verbindungselement eine Zugkraft ausgeübt, wird der zweite Anker in diesen Abschnitt der zweiten Durchführung bewegt und darin vorzugsweise vollständig aufgenommen. In dieser Position ist es nicht möglich, das Verbindungselement aus der zweiten Durchführung zu entnehmen. Das Verbindungselement kann nicht entlang der Längsrichtung der zweiten Durchführung aus dieser herausgezogen werden, da der zweite Anker aufgrund seines Querschnittes nicht durch die zweite Durchführung hindurchpasst. Das Verbindungselement kann auch nicht durch den Schlitz der zweiten Durchführung entfernt werden, da dessen Breite nicht ausreichend, um den zweiten Anker passieren zu lassen. Erst wenn das Verbindungselementes in die entgegengesetzte Richtung bewegt und der zweite Anker aus dem Abschnitt der zweiten Durchführung entfernt wird, kann das Verbindungselement durch den Schlitz der zweiten Durchführung bewegt und so von der Befestigungseinrichtung und damit vom formstabilen Element der orthopädietechnischen Einrichtung entfernt werden.

In einer bevorzugten Ausführungsform ist an dem zweiten Anker die Umlenköse befestigt, wobei der zweite Anker vorzugsweise identisch zu dem ersten Anker ausgebildet ist. In dieser Ausführungsform wird das zweite Ende des Verbindungselementes, an dem sich der zweite Anker befindet, vorzugsweise nicht in eine zweite Durchführung der Befestigungseinrichtung eingelegt oder durch diese hindurch geführt, sondern mit der Umlenköse verbunden. Das Verbindungselement bildet dann keine Schlaufe, sondern verbindet die Befestigungseinrichtung mit der Umlenköse.

Vorzugsweise verfügt die Umlenköse über eine Ösendurchführung, durch die das Verbindungselement geführt ist, wobei die Ösendurchführung vorzugsweise einen Abschnitt aufweist, in dem der zweite Anker vorzugsweise vollständig, zumindest jedoch teilweise, aufnehmbar ist. Der zweite Anker und die Ösendurchführung sind dabei derart ausgebildet, dass das zweite Ende mit dem zweiten Anker nicht entlang der Längsrichtung der Ösendurchführung durch diese gezogen werden kann.

Bevorzugt verfügt die Ösendurchführung über einen Schlitz, dessen Breite größer als der Durchmesser des Verbindungselementes und kleiner als der Durchmesser des zweiten Ankers ist. Die Ösendurchführung wirkt dann wie die erste Durchführung und/oder die zweite Durchführung, wenn diese einen entsprechenden Schlitz aufweisen.

Vorzugsweise verfügt das formstabile Element über mehrere Befestigungseinrichtungen. Diese sind an unterschiedlichen Stellen des formstabilen Elementes oder gegebenenfalls an mehreren formstabilen Elementen der orthopädietechnischen Einrichtung angeordnet. Vorzugsweise verfügt die orthopädietechnische Einrichtung über weniger Gurte und weniger Umlenkösen damit vorzugsweise auch über weniger Verbindungselemente als über Befestigungseinrichtungen. Da das Verbindungselement, durch das die Umlenköse mit dem formstabilen Element der orthopädietechnischen Einrichtung verbunden ist, von der Befestigungseinrichtung entfernt und demontiert werden kann, ist es möglich, die Befestigungseinrichtung, an der das Verbindungselement angeordnet ist, zu wählen und auch nach der Montage zu verändern. Damit kann auf individuelle Wünsche des Trägers der orthopädietechnischen Einrichtung eingegangen werden, um ein möglichst angenehmes Tragegefühl zu erreichen. Zudem kann der Orthopädietechniker auch die Position wählen, die die Wirkungsweise der Einrichtung optimiert und dazu beispielsweise mehrere Positionen am Patienten ausprobieren.

Vorzugsweise sind die Befestigungseinrichtungen äquidistant zueinander angeordnet. Besonders bevorzugt verlaufen die ersten Durchführungen und/oder, sofern vorhanden, zweiten Durchführungen der verschiedenen Befestigungseinrichtungen parallel zueinander.

In einer bevorzugten Ausgestaltung der orthopädietechnischen Einrichtung sind das formstabile Element und wenigstens eine der Befestigungseinrichtungen, vorzugsweise alle Befestigungseinrichtungen in einem additiven Fertigungsverfahren, beispielsweise einem 3-D-Druckverfahren, hergestellt. Verfügt das formstabile Element der orthopädietechnischen Einrichtung nur über eine Befestigungseinrichtung ist diese vorzugsweise einstückig mit dem Rest des formstabilen Elementes ausgebildet. Für bestimmte Anwendungen kann es von Vorteil sein, wenigstens eine Befestigungseinrichtung gemeinsam mit dem Rest des formstabilen Elementes in dem additiven Fertigungsverfahren herzustellen und wenigstens eine Befestigungseinrichtung als separates Bauteil auszubilden. Dieses kann an den Rest des formstabilen Elementes angeklebt oder eingeschweißt werden oder lösbar mit diesem verbunden sein. Auch für additiv gefertigte orthopädietechnische Einrichtungen, die mit wenig Aufwand individualisiert werden können, hat es sich überraschenderweise als vorteilhaft herausgestellt mehrere Befestigungseinrichtungen vorzusehen anstelle bereits im Vorfeld eine optimale Position zu definieren. Die Anpassung am Patienten entscheidend zur Bestimmung der optimalen Position beiträgt.

Vorzugsweise handelt es sich bei der orthopädietechnischen Einrichtung um eine Knöchelorthese mit einem Unterschenkelteil und einem Fußteil. Das Unterschenkelteil ist ausgebildet, um an den Unterschenkel des Trägers angelegt zu werden. Vorzugsweise ist es individuell an den Träger angepasst worden. Das Fußteil des ausgebildet, an den Fuß des Trägers angelegt zu werden. Vorzugsweise ist es individuell an den Träger angepasst worden. Das Fußteil und das Unterschenkelteil sind vorzugsweise gelenkig miteinander verbunden. Die orthopädietechnische Einrichtung in dieser Form verfügt vorzugsweise über mehrere Befestigungseinrichtungen, die entlang der Längserstreckung des Unterschenkelteils angeordnet sind.

Vorzugsweise verfügt das Fußteil über einen formstabilen Anteil, an dem sich eine Befestigungseinrichtung befindet. Diese befindet sich im Fersenbereich des Fußteils an der lateralen Seite. An dieser Stelle ist ein Standardverschluss, wie aus dem Stand der Technik bekannt ist, nur schwer anzuordnen, da der Gurt und/oder dessen Befestigung und/oder die Umlenköse dann am Knöchel des Trägers anliegt. Da im angelegten Zustand der Gurt und damit auch die Umlenköse und deren Befestigung, beispielsweise das Verbindungselement, einer Zugkraft unterworfen sind, wird diese Kraft dann auf den Knöchel übertragen und führt zumindest zu einem unangenehmen Tragegefühl, schlimmstenfalls zu Druckstellen und Schmerzen.. Daher ist es von Vorteil, in dieser Ausgestaltung der Erfindung die Befestigungseinrichtungen distal des Knöchels im Bereich der Ferse des Fußteils anzuordnen, wobei die erste Durchführung und/oder die zweite Durchführung im angelegten Zustand der Orthese in einem Winkel von wenigstens 35°, bevorzugt wenigstens 40° und höchstens 55°, bevorzugt höchstens 50°, besonders bevorzugt in einem Winkel von 45° zur Auflagefläche des Fußteils, insbesondere zum Boden, auf dem sich der Träger der Orthese befindet, erstreckt. Durch ein flexibles Verbindungselement lässt sich der Verlauf der Zugkraft, die durch den Gurt erzeugt und übertragen wird, steuern. Besonders bevorzugt verläuft die erste Durchführung und/oder die zweite Durchführung von einem Bereich hinter den Knöchel bis zu einem Bereich vor dem Knöchel. Die erste Durchführung und/oder die zweite Durchführung können dabei eine Kurve beschreiben und müssen nicht geradlinig ausgebildet sein. Dies ist für alle hier beschriebenen Ausführungsformen zutreffend.

Zudem ist im Bereich des Knöchels häufig das Gelenk der orthopädietechnischen Einrichtung angeordnet, damit das Verbindungselement nicht über diesen Bereich verläuft, kann es durch eine Kanalführung daran vorbeigehführt werden. Auf diese Weise kann die Umlenköse in die optimale Position gebracht werden, ohne dass sich die Befestigungseinrichtung an der gleichen Stelle befinden muss. Dies ist insbesondere von Vorteil, da an der gewünschten Position an der Oberseite der Knöchelorthese häufig vom Orthopädietechniker nachgearbeitet werden muss.

Würde sich in diesem Bereich eine Befestigungseinrichtung befinden, wären die Möglichkeiten zur Nachbearbeitung erheblich eingeschränkt.

Es ist somit ein weiterer Vorteil der vorliegenden Erfindung, dass die Position der Befestigungseinrichtung an dem formstabilen Element und die Position der Umlenköse voneinander entkoppelt sind. Dies ermöglicht es eine orthopädische Einrichtung zu erstellen, die weniger aufbaut und daher besser unter der Kleidung zu tragen ist.

Die Erfindung löst die gestellte Aufgabe zudem durch ein Verfahren zum Herstellen einer orthopädietechnischen Einrichtung der hier beschriebenen Art, bei dem zumindest das formstabile Element mit wenigstens einer Befestigungseinrichtung, vorzugsweise mit allen Befestigungseinrichtungen in einem additiven Fertigungsverfahren hergestellt werden, wobei die Anzahl und/oder die Position und/oder die Orientierung der Befestigungseinrichtungen als Eingabeparameter berücksichtigt werden.

Zum Herstellen des formstabilen Elementes mit der Befestigungseinrichtung oder den Befestigungseinrichtungen in einem additiven Fertigungsverfahren, beispielsweise einem 3-D-Druckverfahren, werden vorzugsweise von einem Benutzer einer entsprechenden Herstellungsvorrichtung, beispielsweise einem 3-D-Drucker, Eingabeparameter abgefragt. Die Herstellungsvorrichtung verfügt zu diesem Zweck vorzugsweise über eine Eingabeeinrichtung, beispielsweise eine grafische Benutzeroberfläche, sodass Eingabeparameter in eine elektrische Steuerung eingegeben und an diese übergeben werden können, die für die Steuerung der Herstellungsvorrichtung zuständig ist. Die Parameter beinhalten beispielsweise die Anzahl der Befestigungseinrichtungen, die in dem additiven Herstellungsverfahren hergestellt werden sollen. Zusätzlich oder alternativ dazu beinhalten die Parameter die Position und/oder die Orientierung der Befestigungseinrichtungen. Die Parameter können in einem elektronischen Datenspeicher, auf den die elektrische Steuerung, die die Herstellungsvorrichtung steuert, Zugriff hat, hinterlegt sein. Alternativ oder zusätzlich dazu werden sie über die Eingabeeinrichtung eingegeben.

Die Position und/oder Orientierung einer Befestigungseinrichtungen kann beispielsweise individuell von einem Benutzer der Herstellungsvorrichtung vorgegeben werden. Alternativ dazu könne lediglich Bereiche des herzustellenden formstabilen Elementes vorgegeben werden, in denen eine bestimmte Anzahl von Befestigungseinrichtungen, die individuell ausgewählt werden oder vorgegeben sein können, angeordnet ist. Vorzugsweise können Parameter der Anordnung vorgegeben, sodass beispielsweise ausgewählt werden kann, dass die Befestigungseinrichtungen äquidistant angeordnet und/oder parallel ausgerichtet werden können.

Mithilfe der beiliegenden Figuren werden nachfolgend einige Ausführungsbeispiele der vorliegenden Erfindung näher erläutert.

Es zeigen
Figuren 1 bis 4 - schematische Darstellungen eines formstabilen Elementes mit wenigstens einer Befestigungseinrichtungen,
Figuren 5 bis 7 - schematische Darstellungen verschiedener Umlenkösen,
Figuren 8 bis 11 - schematische Darstellungen verschiedener Festigungseinrichtungen,
Figuren 12 bis 14 - schematische Darstellungen verschiedener Elemente gemäß einem weiteren Ausführungsbeispiel der vorliegenden Erfindung,
Figuren 15 bis 17 - schematische Darstellungen verschiedener Elemente gemäß einem weiteren Ausführungsbeispiel der vorliegenden Erfindung und
Figur 18 - eine Darstellung einer orthopädietechnischen Einrichtung gemäß einem Ausführungsbeispiel der vorliegenden Erfindung.

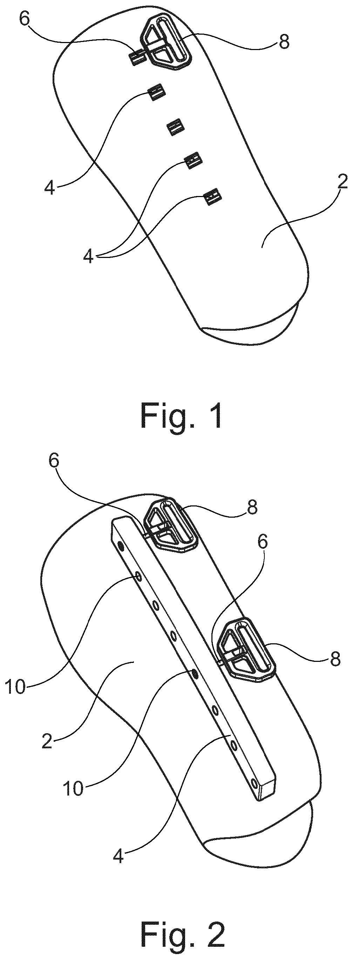

Figur 1 zeigt ein formstabiles Element 2 einer orthopädietechnischen Einrichtung gemäß einem ersten Ausführungsbeispiel der vorliegenden Erfindung. Es handelt sich um eine Unterschenkelschale, die beispielsweise für eine Knöchelorthese verwendet und an einem Unterschenkel eines Trägers der Orthese angeordnet werden kann. Das formstabile Element 2 verfügt über mehrere Befestigungseinrichtungen 4, die nach außen von dem Grundkörper des formstabilen

Elementes 2 hervorstehen. Die Form und Ausgestaltung der Befestigungseinrichtungen 4 werden weiter unten näher beschrieben. An der obersten dargestellten Befestigungseinrichtungen 4 ist über ein Verbindungselement 6, das in Form einer sehr kurzen Schnur ausgebildet ist, eine Umlenköse 8 angeordnet.

Figur 2 zeigt ein formstabiles Element 2, das ebenfalls eine Mehrzahl von Befestigungseinrichtungen 4 aufweist. Diese sind einstückig miteinander ausgebildet. Jede der Befestigungseinrichtungen 4 verfügt über eine Durchführung 10, wobei die Durchführungen 10 und damit die Befestigungseinrichtungen 4 im gezeigten Ausführungsbeispiel äquidistant angeordnet sind. In Figur 2 sind zwei Umlenkösen 8 vorhanden, die jeweils über ein Verbindungselement 6 mit der Befestigungseinrichtungen 4 verbunden sind. Jedes der Verbindungselemente 6 erstreckt sich durch eine Durchführungen 10, was in Figur 2 jedoch nur schematisch angedeutet ist.

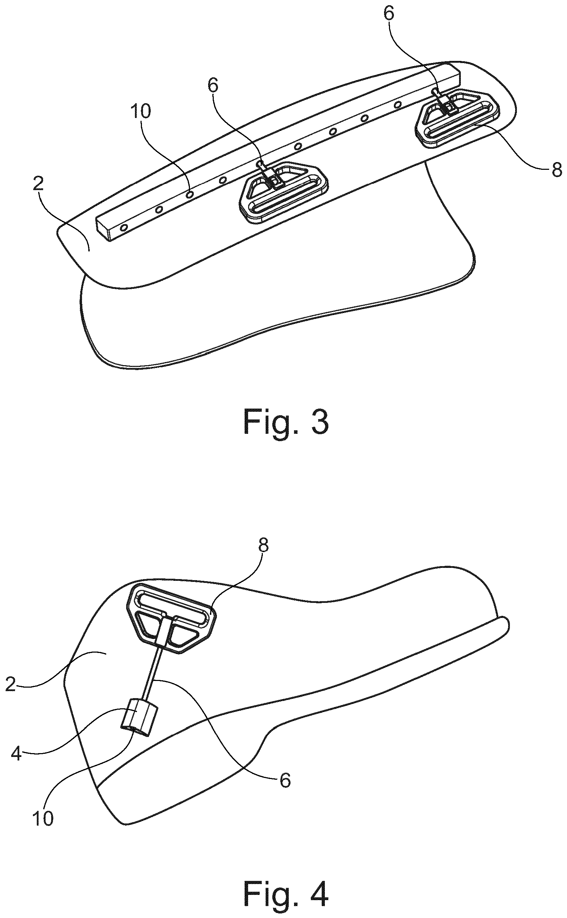

Figur 3 zeigt das formstabile Element 2 aus Figur 2 aus einer anderen Perspektive. In Figur 3 ist gut zu erkennen, dass sich die beiden Verbindungselemente 6, mit denen jeweils eine der beiden Umlenkösen 8 befestigt ist, in eine der Durchführungen 10 erstrecken.

Figur 4 zeigt eine andere Ausgestaltung. Hier ist das formstabile Element 2 Teil eines Fußteils einer Knöchelorthese. Die Umlenköse 8 ist über das Verbindungselement 6 mit einer Befestigungseinrichtungen 4 verbunden, die eine Durchführung 10 aufweist, durch die sich das Verbindungselement 6 erstreckt.

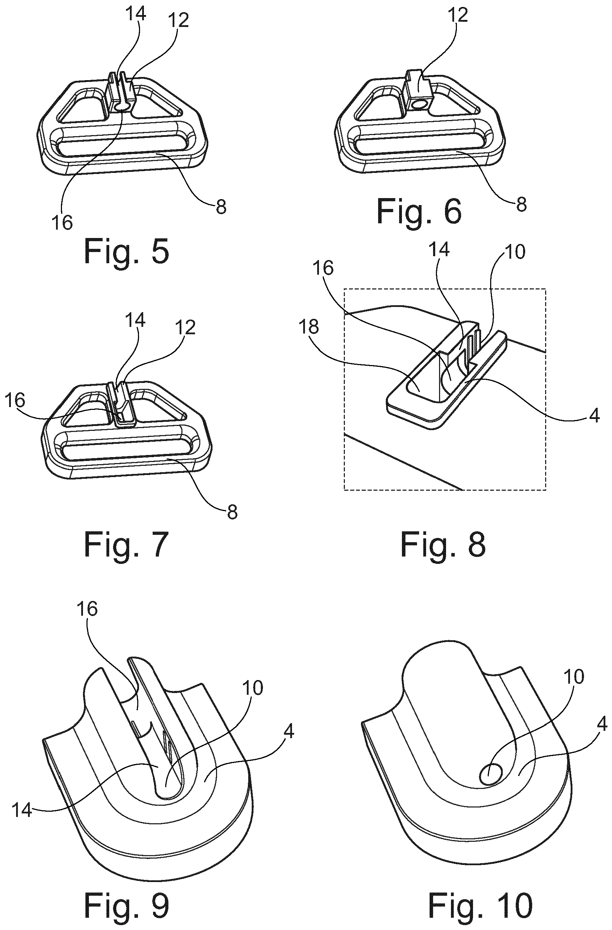

Die Figuren 5 bis 7 zeigen jeweils eine Umlenköse 8, durch die ein Gurt hindurchgeführt und umgelenkt werden kann. Jede der Umlenkösen 8 zeigt eine Ösendurchführung 12, die in den Figuren 5 bis 7 jeweils unterschiedlich ausgebildet ist. In Figur 5 verfügt die Ösendurchführung 12 über einen Schlitz 14, der sich im gezeigten Ausführungsbeispiel über die gesamte Längserstreckung der Ösendurchführung 12 erstreckt. Der Schlitz 14 weist eine Breite auf, die größer ist als ein Durchmesser eines Verbindungselementes 6, das durch die Ösendurchführung 12 hindurchgeführt werden kann. Am in Figur 5 unteren Ende der Ösendurchführung 12 verfügt dieser über einen Abschnitt 16, der einen größeren Durchmesser als der restliche Teil der Ösendurchführung 12 aufweist. Ein Verbindungselement 6, an dessen Ende sich ein Anker befindet und dass durch die Ösendurchführung 12 hindurchgeführt ist, kann in Figur 5 soweit nach oben verschoben werden, bis der Anker in diesem Abschnitt 16 teilweise, vorzugsweise jedoch vollständig aufgenommen ist. Ab diesem Moment ist es nicht möglich, das Verbindungselement 6 weiter in diese Richtung durch die Ösendurchführung 12 hindurch zu ziehen.

Figur 6 zeigt eine ähnliche Form, die sich von der in Figur 5 dargestellten Form der Ösendurchführung 12 lediglich dadurch unterscheidet, dass sie keinen Schlitz aufweist. Figur 7 hingegen zeigt eine Ösendurchführung 12, bei der der Abschnitt 16 deutlich zu erkennen ist. Auch die Ösendurchführung 12 in Figur 7 verfügt über den Schlitz 14, ist also nach oben offen. Im Abschnitt 16, der den im Vergleich zum Rest der Ösendurchführung 12 größeren Querschnitt aufweist, weist auch der Schlitz 14 eine größere Breite auf. Ein Verbindungselement 6, an dessen Ende sich ein Anker befindet, kann also von oben durch den Schlitz 14 in die Ösendurchführung 12 eingelegt werden. Erst wenn eine Zugkraft auch das Verbindungselement 6 wirkt, ist es aufgrund der dabei entstehenden Reibung nicht mehr möglich, dass Verbindungselementes 6 aus der Ösendurchführung 12 zu entfernen.

Die Figuren 8 bis 11 zeigen unterschiedliche Ausführungen einer Befestigungseinrichtung 4. Figur 8 zeigt eine erste Ausgestaltung, bei der die Durchführung 10 einen Schlitz 14 aufweist, also nach oben offen ausgebildet ist. Man erkennt im Innern der Durchführung 10 einen Abschnitt 16, der einen größeren Querschnitt als der verbleibende Teil der Durchführung 10 aufweist. Dieser Abschnitt ist eingerichtet, einen Anker, der sich an einem Ende eines Verbindungselementes 6 befindet, vollständig aufzunehmen. Um diesen Anker in dem Abschnitt 16 positionieren zu können, verfügt die Befestigungseinrichtung 4 über eine Einführöffnung 18, deren Abmessungen so groß sind, dass der Anker vollständig aufgenommen werden kann.

Figur 9 zeigt eine Befestigungseinrichtung 4, bei der die Durchführung 10 den bereits besprochenen Schlitz aufweist. Man erkennt ihm in Figur 9 hinteren Teil der Befestigungseinrichtung 4 den Abschnitt 16 mit dem größeren Querschnitt. Die in Figur 10 gezeigte Befestigungseinrichtung 4 unterscheidet sich von der Befestigungseinrichtung 4 aus Figur 9 dadurch, dass die Durchführung 10 keinen Schlitz aufweist.

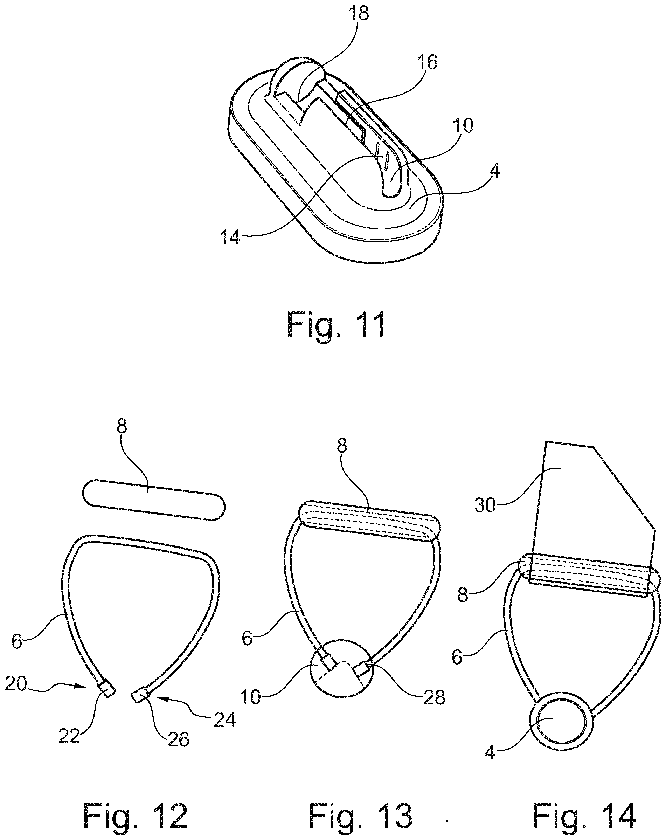

In Figur 11 ist eine Befestigungseinrichtung 4 dargestellt, die in Funktionsweise und Ausgestaltung der Befestigungseinrichtung 4 aus Figur 8 sehr nahekommt. Sie ist lediglich als erhabenes Element ausgebildet und wird folglich auf einen Grundkörper eines formstabilen Elementes aufgesetzt. Die Durchführung 10 verfügt über den Schlitz 14 und im hinteren Bereich ist die Einführöffnung 18 dargestellt, durch die ein Anker in die Befestigungseinrichtung 4 und dann in den Abschnitt 16 eingeführt werden kann.

Die Figuren 12 bis 14 zeigen schematisch eine andere Ausgestaltung. Figur 12 zeigt zunächst ein Verbindungselement 6, dass ein erstes Ende 20 mit einem ersten Anker 22 und ein zweites Ende 24 mit einem zweiten Anker 26 aufweist. Figur 12 zeigt zudem eine andere Form der Umlenköse 8, die in Form eines Schlauches ausgebildet ist. Die beiden Enden 20, 24 des Verbindungselementes 6 sind eingerichtet, mit einer Befestigungseinrichtung 4 zusammenzuwirken.

Dies ist in Figur 13 dargestellt. Das Verbindungselement 6 erstreckt sich durch die Umlenköse 8, was durch die gestrichelten Linien dargestellt ist. Die beiden Enden 20, 24 des Verbindungselementes 6 sind in eine erste Durchführung 10 und in eine zweite Durchführung 28 eingeführt, sodass das Verbindungselement 6 eine Schlaufe bildet. Figur 14 zeigt die Situation in einer schematischen Draufsicht. Die Enden 20, 24 des Verbindungselementes 6 sind in der Befestigungseinrichtung 4 angeordnet und nicht zu erkennen. Ein Gurt 30 ist um die Umlenköse 8 herumgelegt.

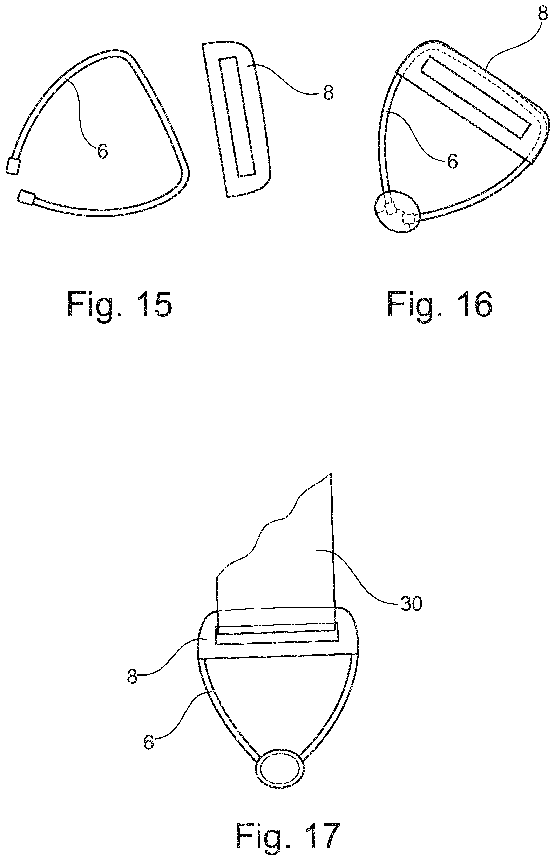

In den Figuren 15 bis 17 erst eine etwas andere Ausgestaltung dargestellt, die sich von der Ausgestaltung in den Figuren 12 bis 14 insbesondere dadurch unterscheidet, dass die Umlenköse 8 nicht nur den Tunnel beinhaltet, durch den das Verbindungselementes 6 hindurchgeführt ist, sondern selbst eine Öse bildet, durch die der Gurt 30 geführt wird.

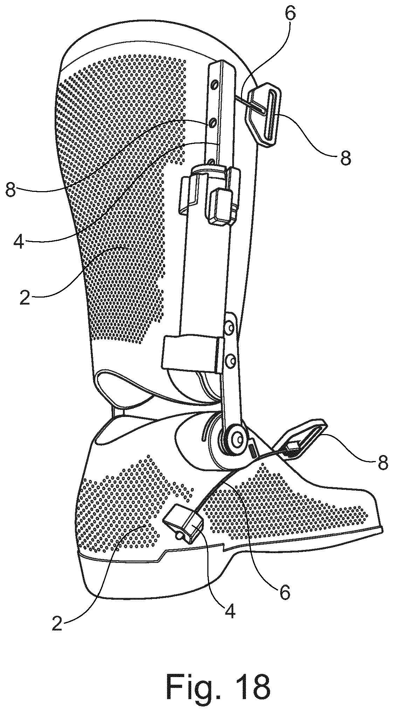

Figur 18 zeigt eine orthopädietechnische Einrichtung in Form einer Knöchelorthese. Sie verfügt über ein erstes formstabiles Element 2 in Form eines Fußteils, das an einen Fuß eines Trägers der orthopädietechnischen Einrichtung angeordnet wird. Sie verfügt zudem über ein zweites formstabiles Element 2 in Form eines Unterschenkelteils, das an einen Unterschenkel des Trägers angelegt werden kann.

Beide formstabilen Elemente 2 haben jeweils wenigstens eine Befestigungseinrichtung 4 mit jeweils einer Durchführung 10. Durch diese ist ein Verbindungselement 6 geführt oder kann ein Verbindungselement 6 geführt werden. An den beiden gezeigten Verbindungselementen 6 ist jeweils eine Umlenköse 8 angeordnet.

### Bezugszeichenliste

2 formstabiles Element
4 Befestigungseinrichtung
6 Verbindungselement
8 Umlenköse
10 Durchführung
12 Ösendurchführung
14 Schlitz
16 Abschnitt
18 Einführöffnung
20 erstes Ende
22 erster Anker
24 zweites Ende
26 zweiter Anker
28 zweite Durchführung
30 Gurt

## Patentansprüche

1. Orthopädietechnische Einrichtung mit einem formstabilen Element (2) zum Positionieren an einem Körperteil und einem Gurt (30) zum Befestigen des formstabilen Elementes (2) an dem Körperteil, wobei der Gurt (30) durch eine Umlenköse (8) geführt ist,
wobei das formstabile Element (2) wenigstens eine Befestigungseinrichtung (4) mit einer Durchführung (10) mit einer Längsrichtung aufweist, entlang der sich ein Verbindungselement (6) durch die Durchführung (10) erstreckt, an dem die Umlenköse (8) befestigt ist, wobei das Verbindungselement (6) ein erstes Ende (20) aufweist, an dem ein erster Anker (22) derart ausgebildet ist, dass das erste Ende (20) mit dem ersten Anker (22) nicht entlang der Längsrichtung durch die Durchführung (10) gezogen werden kann, **dadurch gekennzeichnet, dass** der erste Anker (22) lösbar an dem ersten Ende (20) angeordnet ist.

2. Orthopädietechnische Einrichtung nach Anspruch 1, **dadurch gekennzeichnet, dass** die Durchführung (10) einen Abschnitt (16) aufweist, in dem der erste Anker (22) zumindest teilweise, vorzugsweise vollständig, aufnehmbar ist.

3. Orthopädietechnische Einrichtung nach Anspruch 2, **dadurch gekennzeichnet, dass** die Durchführung (10) einen Schlitz (14) aufweist, dessen Breite größer als der Durchmesser des Verbindungselement (6) und kleiner als der Durchmesser des ersten Ankers (22) ist.

4. Orthopädietechnische Einrichtung nach einem der vorstehenden Ansprüche, **dadurch gekennzeichnet, dass** das Verbindungselement (6) ein zweites Ende (24) aufweist, an dem ein zweiter Anker (26) ausgebildet ist.

5. Orthopädietechnische Einrichtung nach Anspruch 4, **dadurch gekennzeichnet, dass** die Befestigungseinrichtung (4) eine zweite Durchführung (28) mit einer Längsrichtung aufweist, entlang der sich das Verbindungselement (6) erstreckt, wobei der zweite Anker (26) derart ausgebildet ist, dass das zweite Ende (24) mit dem zweiten Anker (26) nicht entlang der Längsrichtung durch die zweite Durchführung (28) gezogen werden kann.

6. Orthopädietechnische Einrichtung nach Anspruch 5, **dadurch gekennzeichnet, dass** die zweite Durchführung (28) einen Schlitz (14) aufweist, dessen Breite größer als der Durchmesser des Verbindungselementes (6) und kleiner als der Durchmesser des zweiten Ankers (26) ist.

7. Orthopädietechnische Einrichtung nach einem der Ansprüche 4, 5 oder 6, **dadurch gekennzeichnet, dass** an dem zweiten Anker (26) die Umlenköse (8) befestigt ist, wobei der zweite Anker (26) vorzugweise identisch zu dem ersten Anker (22) ausgebildet ist.

8. Orthopädietechnische Einrichtung nach Anspruch 7, **dadurch gekennzeichnet, dass** die Umlenköse (8) eine Ösendurchführung (12) aufweist, durch die das Verbindungselement (6) geführt ist, wobei die Ösendurchführung (12) vorzugsweise einen Abschnitt (16) aufweist, in dem der zweite Anker (26) zumindest teilweise, vorzugsweise vollständig, aufnehmbar ist.

9. Orthopädietechnische Einrichtung nach Anspruch 8, **dadurch gekennzeichnet, dass** die Ösendurchführung (12) einen Schlitz (14) aufweist, dessen Breite größer als der Durchmesser des Verbindungselementes (6) und kleiner als der Durchmesser des zweiten Ankers (26) ist.

10. Orthopädietechnische Einrichtung nach einem der vorstehenden Ansprüche, **dadurch gekennzeichnet, dass** das formstabile Element (2) mehrere Befestigungseinrichtungen (4) aufweist.

11. Orthopädietechnische Einrichtung nach Anspruch 10, **dadurch gekennzeichnet, dass** die Befestigungseinrichtungen (4) äquidistant zueinander angeordnet sind.

12. Orthopädietechnische Einrichtung nach einem der vorstehenden Ansprüche, **dadurch gekennzeichnet, dass** das formstabile Element (2) und wenigstens eine Befestigungseinrichtung (4), vorzugsweise alle Befestigungseinrichtungen (4), in einem additiven Fertigungsverfahren hergestellt sind.

13. Orthopädietechnische Einrichtung nach einem der vorstehenden Ansprüche, **dadurch gekennzeichnet, dass** die orthopädietechnische Einrichtung eine Knöchelorthese mit einem Unterschenkelteil und einem Fußteil ist, wobei vorzugsweise mehrere Befestigungseinrichtungen (4) entlang einer Längserstreckung des Unterschenkelteils angeordnet sind.

14. Verfahren zum Herstellen einer orthopädietechnischen Einrichtung nach einem der vorstehenden Ansprüche, **dadurch gekennzeichnet, dass** zumindest das formstabile Element (2) mit wenigstens einer Befestigungseinrichtung (4), vorzugsweise mit allen Befestigungseinrichtungen (4) in einem additiven Fertigungsverfahren hergestellt werden, wobei die Anzahl und/oder die Position und/oder die Orientierung der Befestigungseinrichtungen (4) als Eingabeparameter berücksichtigt werden.

## Claims

1. An orthopaedic device with a dimensionally stable element (2) for positioning on a body part and a strap (30) for fastening the dimensionally stable element (2) to the body part, the strap (30) being guided through a deflection eyelet (8), wherein the dimensionally stable element (2) comprises at least one fastening device (4) with a feed-through (10) with a longitudinal direction, along which a connection element (6) extends through the feed-through (10) to which the deflection eyelet (8) is attached, the connection element (6) comprising a first end (20) on which a first anchor (22) is formed in such a way that the first end (20) with the first anchor (22) cannot be pulled through the feed-through (10) along the longitudinal direction, **characterised in that** the first anchor (22) is detachably arranged on the first end (20).

2. The orthopaedic device according to claim 1, **characterised in that** the feed-through (10) features a section (16) in which the first anchor (22) can be at least partially, preferably completely, accommodated.

3. The orthopaedic device according to claim 2, **characterised in that** the feed-through (10) comprises a slit (14), the width of which is greater than the diameter of the connection element (6) and smaller than the diameter of the first anchor (22).

4. The orthopaedic device according to one of the preceding claims, **characterised in that** the connection element (6) has a second end (24) at which a second anchor (26) is formed.

5. The orthopaedic device according to claim 4, **characterised in that** the fastening device (4) features a second feed-through (28) with a longitudinal direction, along which the connection element (6) extends, the second anchor (26) being designed in such a way that the second end (24) with the second anchor (26) cannot be pulled through the feed-through (28) along the longitudinal direction.

6. The orthopaedic device according to claim 5, **characterised in that** the second feed-through (28) comprises a slit (14), the width of which is greater than the diameter of the connection element (6) and smaller than the diameter of the second anchor (26).

7. The orthopaedic device according to one of the claims 4, 5 or 6, **characterised in that** the deflection eyelet (8) is attached to the second anchor (26), the second anchor (26) preferably being identical to the first anchor (22).

8. The orthopaedic device according to claim 7, **characterised in that** the deflection eyelet (8) features an eyelet feed-through (12) through which the connection element (6) is guided, the eyelet feed-through (12) preferably comprising a section (16) in which the second anchor (26) can be at least partially, preferably completely, accommodated.

9. The orthopaedic device according to claim 8, **characterised in that** the eyelet feed-through (12) comprises a slit (14), the width of which is greater than the diameter of the connection element (6) and smaller than the diameter of the second anchor (26).

10. The orthopaedic device according to one of the preceding claims, **characterised in that** the dimensionally stable element (2) comprises multiple fastening devices (4).

11. The orthopaedic device according to claim 10, **characterised in that** the fastening devices (4) are arranged equidistantly to each other.

12. The orthopaedic device according to one of the preceding claims, **characterised in that** the dimensionally stable element (2) and at least one fastening device (4), preferably all fastening devices (4), are produced in an additive manufacturing process.

13. The orthopaedic device according to one of the preceding claims, **characterised in that** the orthopaedic device is an ankle orthosis with a lower leg section and a foot section, wherein multiple fastening devices (4) are preferably arranged along a longitudinal extension of the lower leg section.

14. A method for producing an orthopaedic device according to one of the preceding claims, **characterised in that** at least the dimensionally stable element (2) with at least one fastening device (4), preferably with all fastening devices (4), are produced in an additive manufacturing process, wherein the number and/or the position and/or the orientation of the fastening devices (4) are taken into account as input parameters.

## Revendications

1. Dispositif orthopédique comprenant un élément indéformable (2), destiné à être positionné sur une partie du corps, et une sangle (30) destinée à fixer l'élément indéformable (2) sur la partie du corps, la sangle (30) étant menée à travers un oeillet de renvoi (8), dans lequel
l'élément indéformable (2) comprend au moins un dispositif de fixation (4) muni d'un passage (10) ayant une direction longitudinale le long de laquelle s'étend un élément de liaison (6) à travers le passage (10), auquel est fixé l'oeillet de renvoi (8), l'élément de liaison (6) présentant une première extrémité (20) sur laquelle un premier ancrage (22) est formé de telle sorte que la première extrémité (20) ayant le premier ancrage (22) ne peut pas être tirée à travers le passage (10) le long de la direction longitudinale,
**caractérisé en ce que** le premier ancrage (22) est disposé de manière amovible sur la première extrémité (20).

2. Dispositif orthopédique selon la revendication 1,
**caractérisé en ce que** le passage (10) présente un tronçon (16) dans lequel le premier ancrage (22) peut être logé au moins partiellement, de préférence entièrement.

3. Dispositif orthopédique selon la revendication 2,
**caractérisé en ce que** le passage (10) présente une fente (14) dont la largeur est supérieure au diamètre de l'élément de liaison (6) et inférieure au diamètre du premier ancrage (22).

4. Dispositif orthopédique selon l'une des revendications précédentes, **caractérisé en ce que** l'élément de liaison (6) présente une deuxième extrémité (24) sur laquelle est formé un deuxième ancrage (26).

5. Dispositif orthopédique selon la revendication 4,
**caractérisé en ce que** le dispositif de fixation (4) présente un deuxième passage (28) ayant une direction longitudinale le long de laquelle s'étend l'élément de liaison (6), le deuxième ancrage (26) étant formé de telle sorte que la deuxième extrémité (24) ayant le deuxième ancrage (26) ne peut pas être tirée à travers le deuxième passage (28) le long de la direction longitudinale.

6. Dispositif orthopédique selon la revendication 5,
**caractérisé en ce que** le deuxième passage (28) présente une fente (14) dont la largeur est supérieure au diamètre de l'élément de liaison (6) et inférieure au diamètre du deuxième ancrage (26).

7. Dispositif orthopédique selon l'une des revendications 4, 5 ou 6, **caractérisé en ce que** l'oeillet de renvoi (8) est fixé au deuxième ancrage (26), le deuxième ancrage (26) étant de préférence identique au premier ancrage (22).

8. Dispositif orthopédique selon la revendication 7,
**caractérisé en ce que** l'oeillet de renvoi (8) présente un passage d'oeillet (12) à travers lequel est mené l'élément de liaison (6), le passage d'oeillet (12) présentant de préférence un tronçon (16) dans lequel le deuxième ancrage (26) peut être logé au moins partiellement, de préférence entièrement.

9. Dispositif orthopédique selon la revendication 8,
**caractérisé en ce que** le passage d'oeillet (12) présente une fente (14) dont la largeur est supérieure au diamètre de l'élément de liaison (6) et inférieure au diamètre du deuxième ancrage (26).

10. Dispositif orthopédique selon l'une des revendications précédentes, **caractérisé en ce que** l'élément indéformable (2) comprend plusieurs dispositifs de fixation (4).

11. Dispositif orthopédique selon la revendication 10,
**caractérisé en ce que** les dispositifs de fixation (4) sont disposés à équidistance les uns des autres.

12. Dispositif orthopédique selon l'une des revendications précédentes, **caractérisé en ce que** l'élément indéformable (2) et au moins un dispositif de fixation (4), de préférence tous les dispositifs de fixation (4), sont fabriqués par un procédé de fabrication additive.

13. Dispositif orthopédique selon l'une des revendications précédentes, **caractérisé en ce que** le dispositif orthopédique est une orthèse de cheville ayant une partie de jambe et une partie de pied, de préférence, plusieurs dispositifs de fixation (4) étant disposés le long d'une extension longitudinale de la partie de jambe.

14. Procédé de fabrication d'un dispositif orthopédique selon l'une des revendications précédentes,
**caractérisé en ce qu'**au moins l'élément indéformable (2) et au moins un dispositif de fixation (4), de préférence tous les dispositifs de fixation (4), sont fabriqués par un procédé de fabrication additive, le nombre et/ou la position et/ou l'orientation des dispositifs de fixation (4) étant pris en compte comme paramètres d'entrée.
